# EUROPEAN PATENT SPECIFICATION

(11) **EP 1 861 020 B1**
(45) Date of publication and mention of the grant of the patent: **21.01.2009**
(21) Application number: 06737469.4
(22) Date of filing: 08.03.2006
(51) Int. Cl.: A61B 17/128, A61B 17/122, A61B 17/00, A61B 17/064

(54) **MULTI-CLIP DEVICE**
VORRICHTUNG MIT MEHREREN KLAMMERN
DISPOSITIF MULTI-AGRAFE

(30) Priority: 11.03.2005 US 661288 P
(43) Date of publication of application: 05.12.2007
(73) Proprietor: Wilson-Cook Medical Inc., Winston-Salem, NC 27105-4191 (US)
(72) Inventor: SURTI, Vihar, c., Winston-salem, North Carolina 27016 (US); ERICKSON, Leonard, Lewisville, North Carolina 27023 (US)
(74) Representative: Garratt, Peter Douglas
(86) International application number: PCT/US2006/008301
(87) International publication number: WO 2006/098994

(56) References cited:
- EP-A- 1 493 392
- US-A- 5 366 459
- US-A1- 2002 133 178

## Description

### TECHNICAL FIELD

The present invention relates to a multi-clip device, and more specifically to a device for delivering a plurality of clips that can be used to cause hemostasis of blood vessels along the gastrointestinal tract, or that can be used as an endoscopic tool for holding tissue or the like.

### BACKGROUND OF THE INVENTION

Conventionally, a clip may be introduced into a body cavity through an endoscope to grasp living tissue of a body cavity for hemostasis, marking, and/or ligating. In addition, clips are now being used in a number of applications related to gastrointestinal bleeding such as peptic ulcers, Mallory-Weiss tears, Dieulafoy's lesions, angiomas, post-papillotomy bleeding, and small varices with active bleeding.

Gastrointestinal bleeding is a somewhat common and serious condition that is often fatal if left untreated. This problem has prompted the development of a number of endoscopic therapeutic approaches to achieve hemostasis such as the injection of sclerosing agents and contact thermo-coagulation techniques. Although such approaches are often effective, bleeding continues for many patients and corrective surgery therefore becomes necessary. Because surgery is an invasive technique that is associated with a high mortality rate and many other undesirable side effects, there exists a need for highly effective less invasive procedures.

Mechanical haemostatic devices have been used in various parts of the body, including gastrointestinal applications. Such devices are typically in the form of clamps, clips, staples, sutures, etc. that are able to apply sufficient constrictive forces to blood vessels so as to limit or interrupt blood flow. One of the problems associated with conventional haemostatic devices, however, is that they can only be delivered using rigid shafted instruments via incision or trocar cannula. Moreover, none of the conventional haemostatic devices are strong enough to cause permanent hemostasis.
One proposed solution is described in US202/133178 according to the preamble of claim 1 which shows a device for delivery of a plurality of detachable clips. The clips are located in series in an introducer tube and each clip is engaged with a respective manipulating wire.

### SUMMARY OF THE INVENTION

[0014]Accordingly, the present invention provides a delivery device that is capable of delivering a plurality of detachable clips that can reliably grasp tissue within the body cavity of a patient during a medical procedure without injury.

According to one aspect of the present invention, an exemplary multi-clip device is provided and comprises an introducing tube that is insertable into the body cavity. Disposed within the introducing tube (also referred to as the sheath) is an operating wire (also referred to as a drive cable). The operating wire is independently slideable within the introducing tube. In other words, the operating wire can be advanced and retracted independently of the movement of the introducing tube.

A plurality of detachable clips is disposed within the introducing tube. Each clip has an engagement portion at the proximal end thereof and a plurality of arms extending towards the distal end thereof. The arms are formed of a resilient material and are shaped such that the arms have a tendency to be in an open position towards the distal end of the clip. The engagement portion is configured such that it can be engaged by the distal end of the operating wire or by the closed arms of another clip. In one embodiment the clip comprises a pair of arms, and in another embodiment the clip comprises three of more arms.

An expandable collar is slidably disposed about the arms of the clip and is configured such that when the collar is moved distally over the arms it closes and holds them in a closed position. The collar has an expanding portion that is movable from a first compressed position to second expanded position. When in the first compress position, the collar is slidably disposed within the introducing tube, and when in the second expanded position, the collar may be engaged by the distal end of the introducing tube so that the introducing tube can be used to advance the collar distally relative to the clip.

The clips are arranged inside the introducing tube in a serial fashion. The distal end of the operating wire engages the engagement portion of the proximal most clip. The arms of the proximal most clip engage the engagement portion of the adjacent distally located clip. Additional clips, if included, are connected to each other in the same "head-to-tail" fashion. In other words, each clip is connected to the operating wire via each of the proximally located intervening clips.

To deploy a clip, the operating wire is advanced distally relative to the introducing tube to extend the arms of the distal most clip out of the distal end of the introducing tube. The distal most clip is advanced far enough to allow the expandable portion of the collar to expand to its second configuration. Once the arms of the clip are positioned about the target tissue, the operating wire is retracted in a proximal direction (or the introducing tube is advanced in a distal direction) to engage the collar with the distal end of the introducing tube. Further proximal movement of the operating wire relative to the introducing tube causes the collar to slide over the arms so as to close the arms onto the tissue. The operating wire is then advanced in a distal direction (or the introducing tube is retracted in a proximal direction) to extend the arms of the next proximally located clip out of the distal end of the introducing tube a distance sufficient to open the arms and release the deployed clip. The delivery device can then be used to deploy another clip by following the same procedure.

An exemplary method of delivering a plurality of detachable clips as disclosed above is described. The method includes disposing a plurality of detachable clips within an introducing tube and connected to each other in a serial arrangement, wherein the proximal most clip is connected to an operating wire movably disposed within the introducing tube. The distal end of the introducing tube is positioned near the targeted tissue. The operating wire is then advanced to expose the distal most clip from the introducing tube. Once the exposed clip is position about the targeted tissue, the operating wire is retracted so as to cause the introducing tube to engage an expandable collar slidably attached to the clip and advance the collar over the arms of the clip. Advancement of the collar over the arms of the clip causes the arms to close onto or about the targeted tissue. Thereafter, the operating wire is advanced so as to expose and open the arms of the next proximally located clip, thereby releasing the deployed clip from the delivery device. The above steps are then repeated to deploy one or more additional detachable clips. The delivery device is then retrieved and the deployed clips are left behind.

In an additional aspect of the present invention there is provided a multi-clip device having an integrated flushing feature. The flushing feature includes a port located in the forward portion of the handle. In one embodiment, the port is in fluid communication with the cavity or open volume within the introducing tube. In another embodiment, the port is in fluid communication with a separate lumen extending through the wall of the introducing tube. This cavity or lumen extends forward from the handle to near the distal end of the introducing tube. As a result, the injection of any fluid, such as saline solution, through the port in the handle is directed through the cavity or lumen and out the distal end of the introducing tube. The flushing feature permits the surgical site to be flushed of blood or other bodily fluids prior to and/or while positioning the clip to grasp the targeted tissue.

According to one method of using the flushing feature described above, the distal end of the clip delivery device is first delivered to the surgical site where the targeted tissue is generally located. If it is determined that the targeted tissue is obscured by blood or other bodily fluids, then saline is injected through the port in the handle so as to pass through the cavity or lumen of the introducing tube. The saline exits the cavity or lumen near the distal end of the introducing tube, thereby flushing the area surrounding the target site of any blood or other bodily fluids. Injection of saline through the port is continued or repeated as necessary to flush the surgical site during the treatment.

### BRIEF DESCRIPTION OF THE DRAWINGS

FIG. 1 is an illustration of one embodiment of the multi-clip device according to the present invention.

FIG. 2 is a cross-sectional illustration of the distal portion of the multi-clip device of Fig. 1 before deployment of a detachable clip.

FIG. 3 is a cross-sectional illustration of the distal portion of the multi-clip device of Fig. 1 during a first stage of deployment of a detachable clip.

FIG. 4 is a cross-sectional illustration of the distal portion of the multi-clip device of Fig. 1 during a second stage of deployment of a detachable clip.

FIG. 5 is a cross-sectional illustration of the distal portion of the multi-clip device of Fig. 1 after deployment and release of a detachable clip.

FIG. 6 is a perspective view of a first embodiment of a detachable clip of the present invention.

FIG. 7 is a perspective view of a second embodiment of a detachable clip of the present invention.

FIG. 8 is a perspective view of an embodiment of an expandable collar of the present invention.

### DESCRIPTION OF THE INVENTION

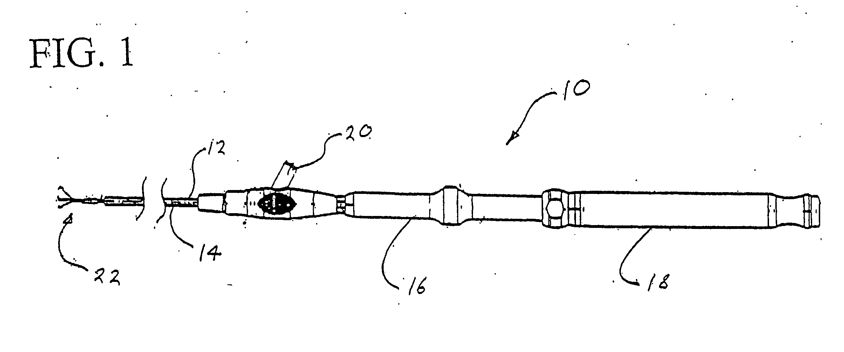

The present invention provides a multi-clip device for grasping tissue or the like. Referring to FIG. 1, an exemplary multi-clip device according to the present invention is shown. The multi-clip delivery device 10 includes an introducing tube 12 that is insertable into the body cavity of a patient. Disposed within the introducing tube 12 (also referred to as the sheath) is an operating wire 14 (also referred to as a drive cable). The operating wire 14 is independently slidable within the introducing tube 12. In other words, the operating wire 14 can be advanced and retracted independently of the movement of the introducing tube 12.

The introducing tube 12 is attached at its proximal end to a forward handle portion 16. The operating wire 14 extends through the forward handle portion 16 and is attached at its proximal end to a rearward handle portion 18, which is disposed proximally of the forward handle portion 16. The rearward handle portion 18 telescopically extends over the proximal portion of the forward handle portion 16. As will be explained in more detail below, longitudinal movement of the operating wire 14 relative to the introducing tube 12 is controlled by longitudinal manipulation of the forward handle portion 16 relative to the rearward handles portion 18.

The forward handle portion 16 also includes a flushing port 20. The flushing port 20 can comprise a standard male or female luer fitting, or any other valve mechanism that permits the injection of fluid there through. The flushing port 20 is in fluid communication with the interior volume of the forward handle portion 16, which in turn is in fluid communication with the cavity or lumen within the introducing tube 12. Accordingly, any fluid injected through the flushing port 20 will necessarily enter the cavity or lumen of the introducing tube 12, and will subsequently exit the cavity near the distal end of the introducing tube 12. In other words, the fluid injected through the flushing port 20 will exit the distal end of the multi-clip device 10.

Alternatively, the cavity or lumen can be disposed within the wall of the introducing tube 12. In other words, the introducing sheath can comprise a separate lumen disposed there through which fluid can be passed along the length thereof. In another alternative embodiment, the lumen can be disposed through the operating wire 14. It should also be understood that the flushing port 20 could be alternatively located on the rearward handle portion 18, or on a portion of the introducing tube 12 distally of any of the handle portions.

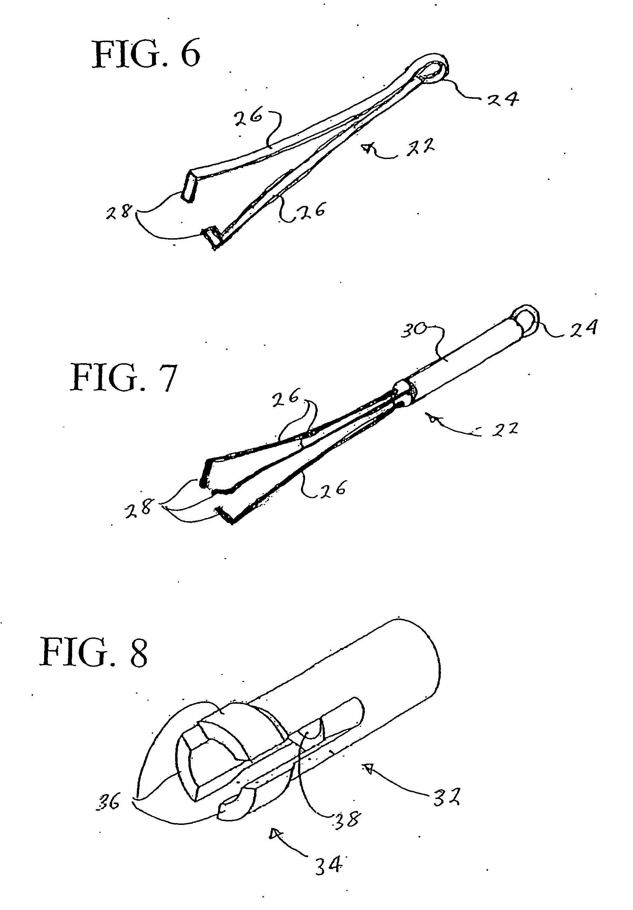

The multi-clip delivery device 10 further includes a plurality of detachable clips 22 disposed within the introducing tube 12. As illustrated in FIGS. 6 and 7, each clip 22 has an engagement portion 24 at the proximal end thereof and a plurality of arms 26 extending towards the distal end thereof. The arms 26 are formed of a resilient material and are shaped such that the arms 26 have a tendency to be in an open position towards the distal end of the clip 22. Each arm 26 includes an inwardly facing prong or tooth 28 which is configured to grasp the target tissue. The teeth 28 preferably overlap with each other when the clip 22 is in the closed position. The length of the individual arms 26 may be different to provide for overlapping of the teeth 28. As will be explained in greater detail below, the engagement portion 24 is configured such that it can be engaged by the distal end of the operating wire 14 or by the closed arms 26 of another clip 22.

In the embodiment illustrated in FIG. 6, the clip 22 comprises a pair of arms 26 formed by bending a single elongate piece of resilient material. The engagement portion 24 is likewise formed by bending the central portion of the elongate piece of resilient material to form a loop.

In the embodiment illustrated in FIG. 7, the clip 22 comprises three arms 26. Each arm 26 is individually formed from a resilient material and affixed to a central tube 30 by any suitable means such as welding or gluing. The engagement portion 24 is likewise individually formed and attached to a proximal end of the central tube 30. The use of three arms allows the clip 22 to grasp the target tissue with minimal, if any, need to rotate the clip 22 into the correct orientation. While three arms are illustrated in this embodiment, it is contemplated that more than three arms may be used.

The clip 22 may be made from any suitable resilient material such as stainless steel, nitinol, plastic, and the like, and is preferably a biocompatible material. The material used for the clip 22 may also be bio-degradable. The use of a bio-degradable material will allow the clip 22 to detach itself from the target tissue after a desired period of time and pass naturally out of the patient's body. In addition, the arms may have a cross-sectional shape that is round, square, triangular, pie-shaped, truncated cone, and the like. For example, the arms 26 of the clip 22 illustrated in FIG. 6 have a rectangular, flat bar-like cross-section, whereas the arms 26 of the clip 22 illustrated in FIG. 7 have a round, wire-like cross-section. A triangular or delta shaped cross-section is particularly advantageous for a clip having three arms because it allows a reduction in the cross-sectional area that the arms occupy within the introducing tube 12, thereby allowing a reduction in the diameter of the introducing tube 12.

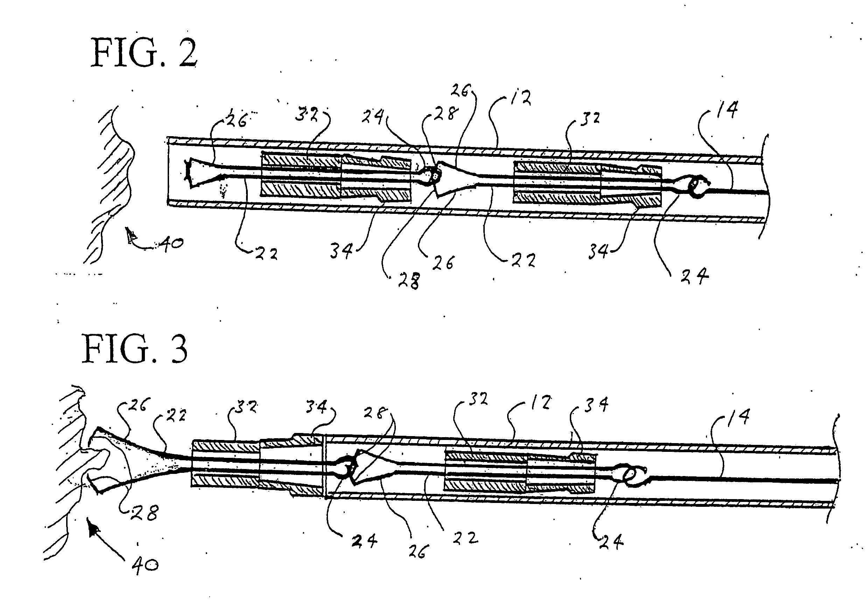

An expandable collar 32 is slidably disposed about the arms 26 of the clip 22 and is configured such that when the collar 32 is moved distally over the arms 26 it closes and holds the arms 26 in a closed position. As illustrated in FIG. 8, the collar 32 has an expanding portion 34 that is movable from a first compressed position to second expanded position. When in the first compress position, the collar 32 may be slidably disposed within the introducing tube 12 (Fig. 2), and when in the second expanded position (FIG. 3), the collar 32 may be engaged by the distal end of the introducing tube 12 so that the introducing tube 12 can be used to advance the collar 32 distally relative to the clip 22.

In the embodiment illustrated in FIG. 8, the expanding portion 34 comprises a plurality of movable arms 36 extending towards the proximal end of the collar 32. The movable arms 36 are biased in an outward transverse direction so as to have an outer diameter that is greater than the inside diameter of the introducing tube 12. However, and as will be explained in greater detail below, the movable arms 36 are compressible to allow the collar 32 to slidably fit within the introducing tube 12. In the alternative, the expanding portion 34 may comprise a resilient material, such as rubber, that is compressible from the expanded position to the compressed position. Such an embodiment would not require any movable components.

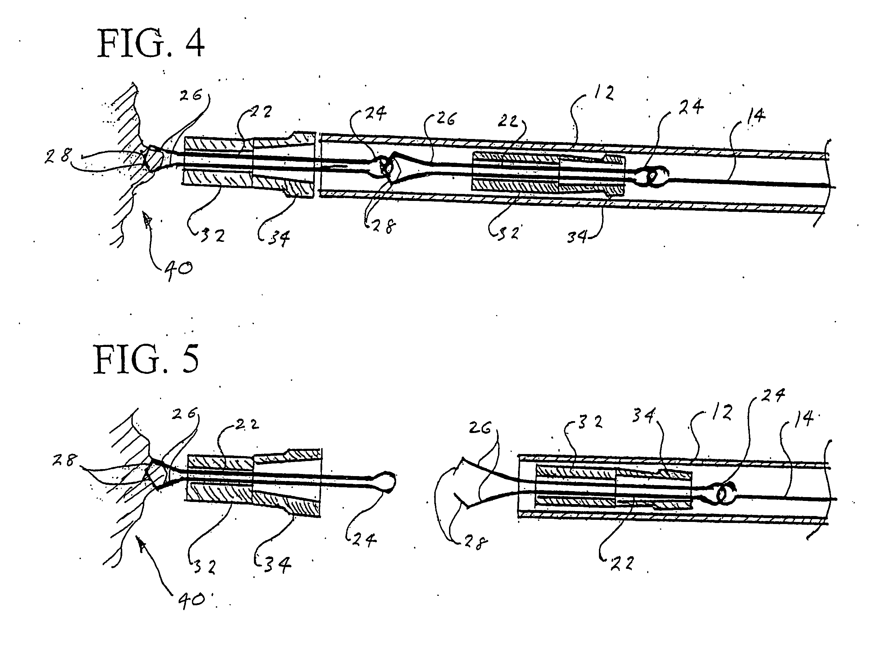

The collar 32 further comprises a central lumen 38 through which the clip 22 is slidably disposed. The central lumen 38 is sized so as to close the arms 26 of the clip 22 as the collar 32 is advanced towards the distal end of the clip 22 (see FIGS. 4 and 5). In other words, the wall portion of the collar 32 that defines the central lumen 38 is configured to engage and overcome the transverse outwardly directed biasing force of the arms 26 so as to push the teeth 28 of the arms 26 into a closed and/or overlapping arrangement as the collar 32 is moved distally relative to the clip 22. The central lumen 38 may also be sized so as to prevent the proximal end of the clip 22, i.e., the engagement portion 24, from passing there through so as to prevent the collar 32 from separating from the clip 22.

The exterior surface of the collar 32 may also be sized and/or configured to allow the passage of fluids around or through the collar 32. As will be explained below, it may be desirable, for example, to pass saline through the introducing tube 12 to flush any blood or bodily fluids away from the part to be treated. Thus, the collar 26 may be sized to provide a gap between the exterior surface of the collar 26 and the interior surface of the introducing tube 12 through which fluids can pass. Alternatively, the collar 26 may include a flow channel or lumen extending there through.

The expandable collar 32 may be made from any suitable resilient material such as plastic, rubber, stainless steel, nitinol, and the like, and is preferably a biocompatible material. The material used for the collar 32 may also be bio-degradable. The expandable collar 32 can be manufactured by any suitable procedure, such as milling (in the case of metal materials) or injection molding (in the case of plastic and rubber materials). While three movable arms 36 are illustrated in the embodiment shown in FIG. 8, it is contemplated any number of movable arms or components may be used. For example, the collar 32 may comprise a single arm or other type of movable or expandable device that can extend outwardly in a transverse direction so as to be engaged by the distal end of the introducing tube 12.

As best seen in FIG. 2, a plurality of clips 22 are arranged inside the introducing tube 12 in a serial fashion. The distal end of the operating wire 14 engages the engagement portion 24 of the proximal most clip 22 (the right-most clip 22 in FIG. 2). The arms 26, and more specifically the teeth 28, of the proximal most clip 22 engage the engagement portion 24 of the adjacent distally located clip 22 (the left-most clip 22 in FIG. 2). The inside surface of the introducing tube 12 maintains the arms 26 in a closed position through or about the engagement portion 24 of the adjacent clip. The expandable collar 32 helps to maintain the position of each clip 22 within the introducing tube 12.

Although the embodiment illustrated in FIG. 2 includes two clips 22, any number of clips 22 can be loaded into the introducing tube 12 of the multi-clip device 10. Additional clips (not shown) may be connected to each other in the same "head-to-tail" fashion as shown in FIG. 2. In other words, each clip 22 is connected to the operating 14 wire via each of the proximally located intervening clips. Thus, each intervening clip 22 functions as an extension of the operating wire 14. This arrangement eliminates the need for separate operating wires connected to each of the individual clips.

The operation of the multi-clip device 10 will be described. Prior to introduction of the device 10 into the patient, a plurality of clips 22 are disposed in the introducing tube 12 and connected to the operating wire 14 as shown in FIG. 2. This may be accomplished by connecting the engagement portion 24 of a first clip 22 to the operating wire 14, and then retracting the operating wire 14 so as to draw the first clip 22 into the introducing tube 12. The expandable collar 32 of the first clip 22 must be compressed so as to allow the collar 32 to be drawn into the introducing tube 12. The engagement portion 24 of a second clip 22 is then connected to the arms 26 of the first clip 22. The operating wire 14 is then further retracted so as to draw the second clip 22, along with its expandable collar 32, into the introducing tube 12. This procedure is repeated until the desired number of clips 22 are loaded into the introducing tube 12 of the device 10.

The introducing tube 12 is then introduced into a body cavity via the channel of an endoscope or similar device that has been previously inserted into the body cavity. While the body cavity is observed via the endoscope, the distal end portion of the outer introducing tube 12 is guided to the part to be treated.

If the part to be treated is obscured by blood or other bodily fluids, then a fluid such as saline is injected through the flushing port 22 on the forward handle portion 16. The saline enters the cavity or lumen within the introducing tube 12 and exits the distal end thereof. The saline floods the area so as to flush any blood or bodily fluids away from the part to be treated. The injection of saline is continued and/or repeated as necessary during the remaining steps of the procedure (described below) so as to keep the area free of blood and other bodily fluids.

Alternatively, a vacuum is applied to the flushing port 22 so as to create suction within the cavity or lumen within introducing tube 12. This suction can be used to remove blood or other bodily fluids from the area surrounding the part to be treated.

To deploy the clip 22, the operating wire 14 is advanced distally relative to the introducing tube 12 to extend the arms 26 of the distal most clip 22 out of the distal end of the introducing tube 12, thereby allowing the arms 26 to assume their expanded, open configuration. As shown in FIG. 3, the distal most clip 22 (the left-most clip 22) is also advanced far enough to allow the expandable portion 34 of the collar 32 to expand to its second expanded configuration. The extended clip 22 can now be positioned about the target tissue 40.

Once the arms 26 of the clip 22 are positioned about the target tissue 40, the operating wire 14 is retracted in a proximal direction (or the introducing tube 12 is advanced in a distal direction) so as to engage the collar 32 with the distal end of the introducing tube12. More specifically, the proximal movement of the operating wire 14 relative to the introducing tube 12 causes the distal end of the introducing tube 12 to engage the expanding portion 34 of the collar 32. As shown in FIG. 4, further proximal movement of the operating wire 14 relative to the introducing tube 12 causes the collar 32 to slide over the arms 26 of the clip 22 so as to close the arms 26 onto the tissue 40. In other words, as the clip 22 is being pulled or drawn proximally into the introducing tube 12, the expanding portion 34 prevents the collar 32 from being likewise pulled or drawn into the introducing tube 12 as the clip 22. The result is that the collar 32 is pushed distally relative to the clip 22.

Once the clip 22 is secured to the target tissue 40 (i.e., deployed), the operating wire 14 is advanced in a distal direction (or the introducing tube 12 is retracted in a proximal direction) to release or detach the deployed clip 22 from the delivery device 10. More specifically, and as shown in FIG. 5, the operating wire 14 is advanced in a distal direction relative to the introducing tube 12 so as to push the next proximally located clip 22 (the right-most clip 22) and collar 32 assembly towards the distal end of the introducing tube 12. Once the arms 26 of the next proximally located clip 22 have been extended out of the distal end of the introducing tube 12 a distance sufficient to open the arms 26, the arms 26 open to disengage from the engagement portion 24 of the deployed clip 22, thereby releasing the deployed clip 22 from the delivery device 10. The delivery device 10 can then be used to deploy another clip 22 by following the same procedure described above.

It should be understood that any number of clips 22 can be initially loaded into the introducing tube 12. Each of these clips 22 would then be available for deployment during the medical procedure without the need to withdraw the multi-clip device 10 for re-loading after each deployment, and without the need to insert additional clip delivery devices.

In another embodiment of the multi-clip device 10, the proximal most clip 22 is permanently affixed to the operating wire 14. In other words, the proximal most clip 22 is not detachable. This non-detachable clip 22 would then be available to function as a forceps for retrieving objects, such as a previously deployed clip 22, or to take tissue samples from the target site. The non-detachable clip could also be used to manipulate and re-position a previously deployed clip 22.

While there have been described what are presently believed to be the preferred embodiments of the invention, those skilled in the art will realize that changes and modifications may be made thereto without departing from the scope of the claims. It is to be understood that the invention can be carried out by specifically different equipment and devices, and that various modifications, both as to the equipment details and operating procedures, can be accomplished without departing from the scope of the claims.

## Claims

1. A multi-clip device for use in endoscopic medical procedures comprising:
a) an introducing tube (12);
b) an operating wire (14) disposed within the introducing tube, the operating wire being advanceable and retractable relative to the introducing tube; and
c) a plurality of detachable clips (22), each clip comprising an engagement portion (24), a plurality of arms (26), and a slidable collar (32) disposed about the plurality of arms, said slidable collar (32) being movable between a first position when said clip is in an open position and a second position when said clip is in a closed position, said slidable collar (32) being configured to cause a distal end of the plurality of arms (26) to be move towards each other when in the second position, the slidable collar (32) comprising an expandable portion (34) being movable between a compressed position when said clip is disposed within the introducing tube (12) and an expanded position when said clip is at least partially disposed beyond a distal end of the introducing tube (12), said expandable portion (34) being configured to be engaged by the distal end of the introducing tube (12) when in the expanded position,
wherein the plurality of detachable clips (22) comprises at least a distal clip and a proximal clip disposed in the introducing tube (12) and arranged in a serial fashion such that the distal clip is disposed distally of the proximal clip,
**characterised in that** the engagement portion (24) of the distal clip is releasably engaged by one or more of the arms (26) of the proximal clip, and the engagement portion (24) of the proximal clip is operably connected to the operating wire (14).

2. The multi-clip device according to claim 1, wherein the expandable portion (34) comprises a plurality of movable arms biased in a transverse outward direction, said movable arms having diameter that is equal or less than an interior diameter of the introducing tube (12) when in the compressed position, and having a diameter that is greater than the interior diameter of the introducing tube (12) when in the expanded position.

3. The multi-clip device according to claim 1, wherein the detachable clips (22) each comprise at least three arms (26).

4. The multi-clip device according to claim 1, wherein each of the plurality of the arms (26) of each of the detachable clips comprises an inwardly directed tooth (28) for engaging a target tissue.

5. The multi-clip device according to claim 1, wherein each of the plurality of the arms (26) of each of the detachable clips is biased in a transverse outward direction.

6. The multi-clip device according to claim 1, wherein the engagement portion (24) of the distal clip (22) comprises a loop configured to be releasably engaged by an inwardly directed tooth (28) disposed on a distal end at least one of the plurality of arms (26) of the proximal clip (22).

7. The multi-clip device according to claim 1, wherein the engagement portion (24) of the proximal clip (22) is affixed to the operating wire (14).

8. The multi-clip device according to claim 7, wherein the proximal clip (22) comprises a forceps.

9. The multi-clip device according to claim 1, wherein the plurality of detachable clips (22) comprises a third clip disposed in the introducing tube (12) and arranged in a serial fashion such that the third clip is disposed distally of the distal clip, further wherein the engagement portion (24) of the third clip is releasably engaged by one or more of the arms of the distal clip.

10. The multi-clip device according to claim 1, wherein the plurality of detachable clips comprises a third clip (22) disposed in the introducing tube (12) and arranged in a serial fashion such that the third clip is disposed proximally of the proximal clip, further wherein the engagement portion (24) of the proximal clip is connected to the operating wire (14) via the third clip.

11. The multi-clip device according to claim 1, wherein the expandable portion (34) of the slidable collar (32) comprises one or more flexible arms that are movable between the compressed position when said clip (22) is disposed within the introducing tube (12) and the expanded position when said clip (22) is at least partially disposed beyond a distal end of the introducing tube (12).

12. The multi-clip device according to claim 1, wherein the slidable collar (32) comprises a lumen (38) through which the arms (26) of the clip (22) are slidably disposed.

13. The multi-clip device according to claim 12, wherein the lumen (38) is defined by an interior surface of the collar (32) that is configured to engage an outer surface of the arms (26) of the clip (22) when the clip is in the closed position.

14. The multi-clip device according to claim 12, wherein the lumen (38) of the collar (32) comprises a cross-sectional area that is smaller than a cross-sectional area of the engagement portion (24) of the clip (22) so as to prevent the collar (32) from being removed from the proximal end of the clip (22).

15. The multi-clip device according to claim 1 further comprising a handle having a first handle portion (16) and a second handle portion (18) movable relative to the first portion, the first handle portion (16) being connected to the introducing tube (12) and the second handle portion (18) being connected to the operating wire (14), wherein manipulation of the first handle portion relative to the second handle portion causes the operating wire to advance or retract relative to the introducing tube.

16. The multi-clip device according to claim 15, wherein the handle further comprises a port (20) configured for the passage of fluids therethrough.

17. The multi-clip device according to claim 16, wherein the port (20) is in fluid communication with the distal end of the introducing tube (12).

18. The multi-clip device according to claim 17, wherein port (20) is in fluid communication with a lumen of the introducing tube (12).

19. The multi-clip device according to claim 17, wherein the slidable collar (32) comprises a pathway (38) for the passage of fluids between the port and the distal end of the introducing tube (12).

## Patentansprüche

1. Multiclip-Vorrichtung zur Verwendung in endoskopischen medizinischen Eingriffen, umfassend:
a) ein Einführungsrohr (12);
b) einen Bedienungsdraht (14), der in dem Einführungsrohr angeordnet ist, wobei der Bedienungsdraht relativ zum Einführungsrohr vorschiebbar und zurückziehbar ist; und
c) eine Vielzahl von lösbaren Clips (22), wobei jeder Clip einen Eingriffsteil (24), eine Vielzahl von Armen (26) und einen verschiebbaren Kragen (32) umfasst, der um die Vielzahl von Armen herum angeordnet ist, wobei der verschiebbare Kragen (32) zwischen einer ersten Position, in der sich der Clip in einer offenen Stellung befindet, und einer zweiten Position, in der sich der Clip in einer geschlossenen Stellung befindet, verschiebbar ist, wobei der verschiebbare Kragen (32) so konfiguriert ist, dass er dazu führt, dass sich jeweils ein distales Ende der Vielzahl von Armen (26) in der zweiten Position aufeinander zu bewegt, wobei der verschiebbare Kragen (32) einen expandierbaren Abschnitt (32) aufweist, der zwischen einer komprimierten Stellung, in der der Clip im Einführungsrohr (12) angeordnet ist, und einer expandierten Stellung, in der der Clip zumindest teilweise über ein distales Ende des Einführungsrohrs (12) hinaus angeordnet ist, verschoben werden kann, wobei der expandierbare Abschnitt (34) so konfiguriert ist, dass er vom distalen Ende des Einführungsrohrs (12) ergriffen wird, wenn er sich in der expandierten Stellung befindet,
worin die Vielzahl von lösbaren Clips (22) zumindest einen distalen Clip und einen proximalen Clip umfasst, die im Einführungsrohr (12) angeordnet sind und so in Reihe angeordnet sind, dass der distale Clip sich distal vom proximalen Clip befindet,
**dadurch gekennzeichnet, dass** der Eingriffsabschnitt (24) des distalen Clips lösbar von einem oder mehr Armen (26) des proximalen Clips ergriffen wird und der Eingriffsabschnitt (24) des proximalen Clips mit dem Bedienungsdraht (14) in Wirkverbindung steht.

2. Multiclip-Vorrichtung nach Anspruch 1, worin der expandierbare Abschnitt (34) eine Vielzahl von beweglichen Armen umfasst, die in einer Auswärtsquerrichtung vorgespannt sind, wobei die beweglichen Arme einen Durchmesser aufweisen, der gleich oder kleiner ist als ein Innendurchmesser des Einführungsrohrs (12) in der komprimierten Stellung, und die einen Durchmesser aufweisen, der größer ist als der Innendurchmesser des Einführungsrohrs (12) in der expandierten Stellung.

3. Multiclip-Vorrichtung nach Anspruch 1, worin die lösbaren Clips (22) jeweils zumindest drei Arme (26) aufweisen.

4. Multiclip-Vorrichtung nach Anspruch 1, worin jeder der Vielzahl von Armen (26) jedes lösbaren Clips einen nach innen gerichteten Zahn (28) zum Eingriff in ein Zielgewebe umfasst.

5. Multiclip-Vorrichtung nach Anspruch 1, worin jeder der Vielzahl von Armen (26) jedes lösbaren Clips in einer Auswärtsquerrichtung vorgespannt ist.

6. Multiclip-Vorrichtung nach Anspruch 1, worin der Eingriffsabschnitt (24) des distalen Clips (22) eine Schlaufe umfasst, die so konfiguriert ist, dass sie lösbar von einem nach innen gerichteten Zahn (28) ergriffen wird, der an einem distalen Ende zumindest eines der Vielzahl von Armen (26) des proximalen Clips (22) angeordnet ist.

7. Multiclip-Vorrichtung nach Anspruch 1, worin der Eingriffsabschnitt (24) des proximalen Clips (22) am Bedienungsdraht (14) befestigt ist.

8. Multiclip-Vorrichtung nach Anspruch 7, worin der proximale Clip (22) eine Zange umfasst.

9. Multiclip-Vorrichtung nach Anspruch 1, worin die Vielzahl von lösbaren Clips (22) einen dritten Clip umfasst, der im Einführungsrohr (12) angeordnet ist und so in Reihe angeordnet ist, dass sich der dritte Clip distal vom distalen Clip befindet, und worin ferner der Eingriffsabschnitt (24) des dritten Clips lösbar von ein oder mehr Armen des distalen Clips ergriffen wird.

10. Multiclip-Vorrichtung nach Anspruch 1, worin die Vielzahl von lösbaren Clips einen dritten Clip (22) umfasst, der im Einführungsrohr (12) angeordnet ist und so in Reihe angeordnet ist, dass sich der dritte Clip proximal vom proximalen Clip befindet, und worin ferner der Eingriffsabschnitt (24) des proximalen Clips über den dritten Clip mit dem Bedienungsdraht (14) verbunden ist.

11. Multiclip-Vorrichtung nach Anspruch 1, worin der expandierbare Abschnitt (34) des verschiebbaren Kragens (32) einen oder mehr flexible Arme umfasst, die zwischen der komprimierten Stellung, in der der Clip (22) im Einführungsrohr (12) angeordnet ist, und der expandierten Stellung, in der der Clip (23) zumindest teilweise über ein distales Ende des Einführungsrohrs (12) hinaus angeordnet ist, bewegt werden kann.

12. Multiclip-Vorrichtung nach Anspruch 1, worin der verschiebbare Kragen (32) ein Lumen (38) umfasst, durch das die Arme (26) des Clips (22) verschiebbar angeordnet sind.

13. Multiclip-Vorrichtung nach Anspruch 12, worin das Lumen (38) von einer Innenfläche des Kragens (32) definiert ist, die so konfiguriert ist, dass sie in eine Außenfläche der Arme (26) des Clips (22) eingreift, wenn der Clip sich in der geschlossenen Stellung befindet.

14. Multiclip-Vorrichtung nach Anspruch 12, worin das Lumen (38) des Kragens (32) einen Querschnittsbereich umfasst, der kleiner ist als ein Querschnittsbereich des Eingriffsabschnitts (24) des Clips (22), so dass verhindert wird, dass der Kragen (32) vom proximalen Ende des Clips (22) entfernt wird.

15. Multiclip-Vorrichtung nach Anspruch 1, ferner umfassend einen Handgriff mit einem ersten Handgriffabschnitt (16) und einem zweiten Handgriffabschnitt (18), der relativ zum ersten Abschnitt bewegbar ist, wobei der erste Handgriffabschnitt (16) mit dem Einführungsrohr (12) verbunden ist und der zweite Handgriffabschnitt (18) mit dem Bedienungsdraht (14) verbunden ist, worin Manipulation des ersten Handgriffabschnitts relativ zum zweiten Handgriffabschnitt dazu führt, dass der Bedienungsdraht relativ zum Einführungsrohr vorgeschoben oder zurückgezogen wird.

16. Multiclip-Vorrichtung nach Anspruch 15, worin der Handgriff ferner eine Öffnung (20) umfasst, die so konfiguriert ist, dass Flüssigkeit durch sie hindurch laufen kann.

17. Multiclip-Vorrichtung nach Anspruch 16, worin die Öffnung (20) mit dem distalen Ende des Einführungsrohrs (12) in Flüssigverbindung steht.

18. Multiclip-Vorrichtung nach Anspruch 17, worin die Öffnung (20) mit einem Lumen des Einführungsrohrs (12) in Flüssigverbindung steht.

19. Multiclip-Vorrichtung nach Anspruch 17, worin der verschiebbare Kragen (32) einen Pfad (38) für den Durchgang von Flüssigkeiten zwischen der Öffnung und dem distalen Ende des Einführungsrohrs (12) umfasst.

## Revendications

1. Dispositif multi-agrafe destiné à être utilisé dans des procédures médicales endoscopiques, comprenant :
a) un tube d'introduction (12) ;
b) un fil fonctionnel (14) disposé dans le tube d'introduction, le fil fonctionnel pouvant être avancé et retiré par rapport au tube d'introduction ; et
c) une pluralité d'agrafes détachables (22), chaque agrafe comprenant une portion d'engagement (24), une pluralité de bras (26) et un collier coulissant (32) disposé autour de la pluralité de bras, ledit collier coulissant (32) pouvant être déplacé entre une première position lorsque ladite agrafe est dans une position ouverte, et une deuxième position lorsque ladite agrafe est dans une position fermée, ledit collier coulissant (32) étant configuré de manière à déplacer les unes des extrémités distales de la pluralité de bras (26) les unes vers les autres lorsqu'il est dans la deuxième position, le collier coulissant (32) comprenant une portion extensible (34) pouvant être déplacée entre une position comprimée lorsque ladite agrafe est disposée dans le tube d'introduction (12), et une position étendue lorsque ladite agrafe est au moins partiellement disposée au-delà d'une extrémité distale du tube d'introduction (12), ladite portion extensible (34) étant configurée de manière à être engagée par l'extrémité distale du tube d'introduction (12) lorsqu'elle est dans la position étendue,
la pluralité d'agrafes détachables (22) comprenant au moins une agrafe distale et une agrafe proximale disposées dans le tube d'introduction (12) et agencées en série de telle sorte que l'agrafe distale soit disposée distalement par rapport à l'agrafe proximale,
**caractérisé en ce que** la portion d'engagement (24) de l'agrafe distale est engagée de manière détachable par un ou plusieurs des bras (26) de l'agrafe proximale, et la portion d'engagement (24) de l'agrafe proximale est connectée fonctionnellement au fil fonctionnel (14).

2. Dispositif multi-agrafe selon la revendication 1, dans lequel la portion extensible (34) comprend une pluralité de bras mobiles sollicités dans une direction transversale vers l'extérieur, lesdits bras mobiles ayant un diamètre qui est inférieur ou égal à un diamètre intérieur du tube d'introduction (12) lorsque la portion extensible est dans la position comprimée, et ayant un diamètre qui est supérieur au diamètre intérieur du tube d'introduction (12) lorsqu'elle est dans la position étendue.

3. Dispositif multi-agrafe selon la revendication 1, dans lequel les agrafes détachables (22) comprennent chacune au moins trois bras (26).

4. Dispositif multi-agrafe selon la revendication 1, dans lequel chacun de la pluralité des bras (26) de chacune des agrafes détachables comprend une dent (28) orientée vers l'intérieur pour s'engager avec un tissu cible.

5. Dispositif multi-agrafe selon la revendication 1, dans lequel chacun de la pluralité des bras (26) de chacune des agrafes détachables est sollicité dans une direction transversale vers l'extérieur.

6. Dispositif multi-agrafe selon la revendication 1, dans lequel la portion d'engagement (24) de l'agrafe distale (22) comprend une boucle configurée de manière à être engagée de manière détachable par une dent (28) orientée vers l'intérieur disposée sur une extrémité distale d'au moins l'un de la pluralité de bras (26) de l'agrafe proximale (22).

7. Dispositif multi-agrafe selon la revendication 1, dans lequel la portion d'engagement (24) de l'agrafe proximale (22) est fixée au fil fonctionnel (14).

8. Dispositif multi-agrafe selon la revendication 7, dans lequel l'agrafe proximale (22) comprend un forceps.

9. Dispositif multi-agrafe selon la revendication 1, dans lequel la pluralité d'agrafes détachables (22) comprend une troisième agrafe disposée dans le tube d'introduction (12) et agencée en série de telle sorte que la troisième agrafe soit disposée distalement par rapport à l'agrafe distale, et dans lequel la portion d'engagement (24) de la troisième agrafe est en outre engagée de manière détachable par un ou plusieurs des bras de l'agrafe distale.

10. Dispositif multi-agrafe selon la revendication 1, dans lequel la pluralité d'agrafes détachables comprend une troisième agrafe (22) disposée dans le tube d'introduction (12) et agencée en série de telle sorte que la troisième agrafe soit disposée proximalement par rapport à l'agrafe proximale, et dans lequel la portion d'engagement (24) de l'agrafe proximale est en outre connectée au fil fonctionnel (14) par le biais de la troisième agrafe.

11. Dispositif multi-agrafe selon la revendication 1, dans lequel la portion extensible (34) du collier coulissant (32) comprend un ou plusieurs bras flexibles qui peuvent être déplacés entre la position comprimée lorsque ladite agrafe (22) est disposée dans le tube d'introduction (12) et la position étendue lorsque ladite agrafe (22) est au moins partiellement disposée au-delà d'une extrémité distale du tube d'introduction (12).

12. Dispositif multi-agrafe selon la revendication 1, dans lequel le collier coulissant (32) comprend une lumière (38) à travers laquelle les bras (26) de l'agrafe (22) sont disposés de manière coulissante.

13. Dispositif multi-agrafe selon la revendication 12, dans lequel la lumière (38) est définie par une surface intérieure du collier (32) qui est configurée de manière à s'engager avec une surface extérieure des bras (26) de l'agrafe (22) lorsque l'agrafe est dans la position fermée.

14. Dispositif multi-agrafe selon la revendication 12, dans lequel la lumière (38) du collier (32) comprend une surface en section transversale qui est plus petite qu'une surface en section transversale de la portion d'engagement (24) de l'agrafe (22) de manière à empêcher le collier (32) d'être enlevé de l'extrémité proximale de l'agrafe (22).

15. Dispositif multi-agrafe selon la revendication 1, comprenant en outre une poignée ayant une première portion de poignée (16) et une deuxième portion de poignée (18) déplaçable par rapport à la première portion, la première portion de poignée (16) étant connectée au tube d'introduction (12) et la deuxième portion de poignée (18) étant connectée au fil fonctionnel (14), la manipulation de la première portion de poignée par rapport à la deuxième portion de poignée provoquant l'avance ou le retrait du fil fonctionnel par rapport au tube d'introduction.

16. Dispositif multi-agrafe selon la revendication 15, dans lequel la poignée comprend en outre un orifice (20) configuré pour permettre le passage de fluides à travers lui.

17. Dispositif multi-agrafe selon la revendication 16, dans lequel l'orifice (20) est en communication fluidique avec l'extrémité distale du tube d'introduction (12).

18. Dispositif multi-agrafe selon la revendication 17, dans lequel l'orifice (20) est en communication fluidique avec une lumière du tube d'introduction (12).

19. Dispositif multi-agrafe selon la revendication 17, dans lequel le collier coulissant (32) comprend un chemin (38) pour le passage de fluides entre l'orifice et l'extrémité distale du tube d'introduction (12).
